# EUROPEAN PATENT APPLICATION

(11) **EP 4 691 240 A1**
(43) Date of publication of application: **11.02.2026**
(21) Application number: 24777341.9
(22) Date of filing: 26.03.2024
(51) Int. Cl.: A01N 43/38, A01N 43/40, A01N 43/653, A01N 33/04, A01H 4/00, A01G 22/55, A01G 24/20, A01P 21/00

(54) **SUPPLEMENT AND NUTRITIVE SUBSTRATE COMPOSITIONS, SYNTHETIC SEED, METHOD FOR CULTIVATING PLANTS, AND, USE OF A SUPPLEMENT COMPOSITION**

(30) Priority: 31.03.2023 BR 102023006170
(71) Applicant: CTC - Centro de Tecnologia Canavieira S.A., 13400-970 Piracicaba - SP (BR)
(72) Inventor: PRUDENTE, Debora, de Oliveira, 13400-970 Piracicaba, SP (BR); CASTELLANI, Luciana, Goncalves, Chaves, 13400-970 Piracicaba, SP (BR)
(74) Representative: Elzaburu S.L.P.
(86) International application number: PCT/BR2024/050118
(87) International publication number: WO 2024/197369

(57) **Abstract**

The present invention relates to the field of biotechnology. More precisely, a gelatinous nutrient substrate containing at least meta-topolin and melatonin and the method of its production are described. An artificial seed is also described, which comprises a container filled with a nutrient substrate comprising at least melatonin and meta-topolin into which a plant propagule is placed. The invention also describes a method of *in vitro* plant hardening, in which meristematic and/or embryonic tissues grown *in vitro* are placed in a nutrient substrate comprising at least meta-topolin and melatonin with subsequent exposure to the stress conditions commonly found in the field, allowing the plant propagule to develop into an adult plant. Preferably, the meristematic and/or embryonic plant tissues used are those from plant species that can be propagated vegetatively.

## Description

### Technical Field

The present invention relates to the field of biotechnology. More precisely, a nutrient substrate is described which is composed of a basal culture medium for growing plants and at least one supplementation composition comprising no less than one cytokinin and melatonin. An artificial seed is also described as comprising a container filled with a nutritious substrate into which a plant propagule is placed for germination and generation of a new adult plant under *ex vitro* environmental conditions found in the field. The synthetic seeds produced with the nutrient substrate of the present invention can be planted in a greenhouse or in the field in full sun. The invention also describes a plant cultivation method in which plant propagules from *in vitro* cultivation are placed in the nutrient substrate for direct transition to the *ex-vitro* environment, where they will be exposed to stress conditions commonly found in the field.

### Description of the State of the Art

Plant tissue culture techniques have been widely used, not only for commercial plant propagation, but also for plant genetic manipulation, through, for example, genetic transformation, mutagenesis and gene editing methods, in addition to being important tools for conventional plant breeding and seedling sanitation, enabling healthier plantations.

These tissue culture systems are generally referred to as micropropagation systems where plant tissues are grown *in vitro* in suitable culture media from which they multiply and regenerate into mature plants. Micropropagation systems make it possible to maintain the sustained production and quality of plant material for commercial plantations, for example. The propagation and regeneration of plants by tissue culture techniques is widely known in the state of the art and numerous publications suggest techniques and methods for micropropagation of a wide variety of plant species, with successful reports for *in vitro* micropropagation via organogenesis or somatic embryogenesis. In the field, micropropagated sugarcane plants have shown good results in terms of tillering and productivity, for example.

Despite reports of success, the use of these techniques on an industrial scale is an activity that still presents numerous challenges, and there is no consensus even on the parameters that should be observed when defining a process for a particular species.

The state of the art is contradictory when it comes to defining the best practices applicable to plant tissue culture, especially in crops involving species with such complex genomes as sugar cane. It is common for genotype-specific protocols to be defined and for new protocols to be optimized/developed with each new variety cultivated. Thus, *in vitro* cultivation today is still an empirical process in which the following conditions are tested minimally for each species, or even for each variety (genotype) within the same species: (I) different *in vitro* cultivation techniques (organogenesis, direct and indirect somatic embryogenesis); (II) source of explant; (III) composition of the culture medium (vitamins, carbon sources and phytoregulators); (IV) hormonal balance; and (V) environmental conditions (Peres, 2002).

Regarding item (IV) mentioned above, there are several hormones and phytoregulators known to the state of the art. Once the roles of these compounds in each of the stages of micropropagation were discovered, countless possibilities opened up for controlling, optimizing and developing new processes. However, although much knowledge has been generated since then, the type of compound, its concentration, combination, and time of use are still the subject of much current study and development.

For example, the exogenous application of cytokinins is widely used to stimulate the productive response of plants and promote their growth under adverse environmental conditions (Marenco & Lopes, 2007). Another important function of the exogenous application of cytokinins is the delay in plant senescence through the accumulation and maintenance of photosynthetic pigments in plants (Van Staden et al. 1988). Cytokinins have specific effects of increasing the rate of amino acid incorporation, delaying senescence and increasing nutrient absorption (Salisbury & Ross, 1992). They have been used in various crops to accelerate growth rates, induce root formation, prevent fruit drop and adjust the ratio of male to female flowers in inflorescences (Kumar et al., 2011).

However, the use of cytokinins alone is not enough to guarantee the development and regeneration of a plant explant and/or propagule into an adult plant, as other stimuli must also be considered, especially when the seedling from the explant will be subjected to the abiotic stresses of the field directly from *in vitro* cultivation. In addition, there are several cytokinins involved in the development processes of a plant, making it necessary to choose them correctly, both qualitatively and quantitatively, in isolation or in combination.

Thus, the present application teaches a new plant cultivation composition comprising a combination of phyto-regulators and other compounds whose purpose is to provide the nutritional and stimuli conditions necessary for an explant and/or plant propagule to acquire metabolic and physiological characteristics capable of stimulating robust when it is directly placed in *ex vitro* conditions in the field.

The state of the art documents that are closest to the invention claimed are briefly presented below.

Document US2018206427 (A) discloses a plant propagation system based on detachable supports that can be used for micropropagation of plants, in which they are placed in liquid media containing plant development stimulating compounds such as meta-topolin.

Publication WO13016198 (A1) refers to compositions, methods, and systems for plant propagation, specifically bamboo. In media for root development and plant maintenance, cytokinins other than thidiazuron (TDZ) are selected, for example, from the group consisting of N6-benzylaminopurine (BAP), meta-topolin (mt), zeatin, zeatin riboside, dihydrozeatin, kinetin, isopentenyladenine, adenine hemisulfate and the like.

Nawaz *et al.* (2016) suggests the possibility of using melatonin for other crops with potential in the seed industry, especially in the exogenous application of this compound in fruits, seeds, and even roots. However, the study does not disclose or suggest the combination of melatonin and meta-topolin, much less in the concentrations described in the present invention.

### Brief Description of the Invention

The present invention describes a supplementation composition comprising the combination of meta-topolin and melatonin. This composition can be added to the nutrient substrates used to grow explants and/or plant propagules, whether or not they have been previously grown *in vitro.* This nutrient substrate is made up of a basal composition comprising all the essential nutritional sources for growing vegetables, such as basal salts, vitamins, carbon sources, antioxidants, among others, together with one or more phyto-regulators and plant hormones. The supplementation composition of the present invention may comprise other phytoregulators and plant hormones as well, in addition to an amino acid composition and a silicon source.

The supplementation composition of the present invention promotes higher survival rates for explants and/or plant propagules when they are passed directly to *ex vitro* conditions in the field, even under abiotic stresses.

The action of the supplementation composition is based on the redirection of carbon skeletons to accelerate metabolism and growth, with subsequent enzymatic/protein stabilization under stress, delaying senescence, increasing photosynthetic pigment content, inducing the expression of heat shock proteins and promoting cell wall structuring in roots and leaves. These characteristics are desirable because they increase survival rates during the acclimatization phase of plants grown under the action of the supplementation composition of the present invention, because during this phase there is a greater loss of water, due to the low functionality of the stomata and the thin layer of epicuticular wax.

In a first aspect, the present invention provides a supplementation composition characterized by comprising a combination of meta-topolin and melatonin. In more detail, the concentration of melatonin in the supplement composition is characterized as being from 5 to 25 uM, preferably from 10 uM to 15 uM, even more preferably the concentration of melatonin is 10uM. Similarly, the concentration of meta-topolin is characterized by being from 3.5 to 7uM, preferably from 4 to 6 uM, even more preferably the concentration of meta-topolin is 5 uM.

In addition, the supplement composition comprises L-alanine, L-proline and glycine and potassium silicate.

In a second aspect, the present invention provides a nutritive substrate composition, characterized in that it comprises a supplementation composition and a basal composition for growing plants.

Preferably, the basal composition of plant cultivation is characterized by comprising basal salts, vitamins, antioxidant agents, sugars, adsorbing agents and gelling agents.

More preferably, the basal composition of plant cultivation is characterized by the basal salts are Hoagland's salt or MS salts; the vitamins are the B5G formulation; the antioxidants are citric and ascorbic acids and polyvinylpyrrolidone (PVP); the sugars are sucrose; the adsorbing agents are activated charcoal; the adsorbing agents are activated charcoal; and the gelling agents are agar and pigskin gelatine.

Yet a third aspect of the present invention relates to a synthetic seed, characterized in that it comprises (a) a container filled with a nutritive substrate composition, as defined above; (b) a plant propagule or plant explant inserted into the nutritive substrate inside the container of step (a).

Preferably, the propagule or plant explant are somatic embryos, zygotic embryos, meristematic tissues, true seeds and/or a totipotent plant explant.

Also, preferably, the plant species can be selected from: wheat, barley, corn, rice, oats, forage grasses, peat, miscanthus, sugar cane, beans, soybeans, peas, tomatoes, rapeseed, grapes, potatoes, pineapples, strawberries, oranges, cassava, tobacco and ornamental species. In particular, the plant species is a monocot, more preferably sugar cane.

A fifth aspect of the present invention relates to a plant cultivation method which is characterized by comprising (a) the *in vitro* cultivation of a plant explant for the production of a plant propagule; (b) inserting the plant propagule of step (c) into the nutrient substrate as defined above; (c) subjecting the nutrient substrate of step (b) to *ex vitro* field conditions; and (d) regenerating an adult plant.

Finally, this invention provides a sixth aspect which relates to the use of a supplementation composition, as defined above, characterized in that it is for making a nutritious substrate for growing plants.

### Short Description of the Figures

Figure 1 is the result of the germination percentage of somatic embryo clusters (ES clusters) after 21 days of in vitro cultivation (A); shoot length (cm) (B); and leaf area (cm2) (C).
Figure 2 is the visual aspect of the tray from treatment 1 (A); plugs from treatment 1 (B); treatment 2 (C); plugs from treatment 2 (D); treatment 3 (E); plugs from treatment 3 (F); treatment 4 (G); plugs from treatment 4 (H) after 21 days of in vitro cultivation.
Figure 3 is the relationship between the treatments and the leaf and root area (cm2) of somatic embryo clusters after 21 days of *in vitro* cultivation.
Figure 4 shows the visual appearance of the somatic embryo aggregates in the nutrient substrate of the present invention supplemented with the supplementation composition of treatment 13 after 7 days (A); 14 days (B); 21 days with evidence of leaf area (C); and 21 days with evidence of root area (D).
Figure 5 shows shoot height (cm) (A); leaf area (cm²) (B); photosystem II quantum yield (Fv/Fm) (C) and pigment quantification (chlorophyll *a*; chlorophyll b and carotenoids) (D) 30 days after planting the container. Means followed by the same letters do not differ according to the Scott-Knott test (p ≤ 0, 05).
Figure 6 is the percentage survival of plants from somatic embryo clusters at 21, 30, 45, 60 and 90 days of *ex vitro* cultivation in the field.
Figure 7 shows the survival rate (%) of ES clusters after 21, 30, 45 and 60 days after planting in the field (DAP).

### Detailed Description of the Invention

The growth of plants, organs, tissues and plant cells depends on the development of optimized culture media for the perfect interaction of components such as carbon source, nutrients, minerals, nitrogen sources and growth regulators, which enable the maintenance and development of these tissues into new adult plants.

The types of components in the growing medium and their concentration can vary according to the purpose of the crop and there is no consensus in the literature on the best composition for each plant species and purpose.

For example, in plant cultivations where you want to micropropagate a vegetatively reproducing plant species, you would preferentially cultivate a plant explant in culture media containing dedifferentiation stimuli to generate totipotent structures such as callus and/or somatic embryos and then cultivate these tissues under differentiation stimuli into a new plant.

Commonly, in indirect somatic embryogenesis processes, in which the explant is first transformed into a callus in order to be differentiated into a somatic embryo for regeneration, the induction of callogenesis can occur through the phytoregulator 2,4-dichlorophenoxyacetic acid (2,4-D; auxin; WO9001058; Guiderdoni & Demarly, 1988, Plant Cell Tiss. Organ Cult. 90: 71; Ali et al., 2007, Pak. J. Bot. 39: 1961). The 2,4-D is also the phytoregulator of choice for direct somatic embryogenesis processes, in which the explant does not go through the callus phase (Snyman et al., 2001, Acta Hortic 56: 105; Franklin et al., 2006, Plant Growth Regul. 50: 111; Behera & Sahoo, 2009, Nat. Sci. 7: 1), being used alone and often in combinations with other hormonal agents (cytokinins such as kinetin and BAP, IAI and GA₃). Different concentrations of 2,4-D have been reported, and it is a hormonal agent used at different stages of the process, from the induction of callogenesis to the beginning of the somatic embryo maturation stage (Naz et al., 2008; Jahangir et al., 2010). There is still no consensus on how to use this hormonal agent, and numerous variations have been observed, including genotype dependence (Ho&Vasil, 1983; Brisibe et al., 1994, Jiménez, 2001).

In addition to the auxin 2,4-D, other hormones are also frequently used in *in vitro* micropropagation processes. Among them, naphthaleneacetic acid (NAA) (Irvine et al., 1991, Plant Cell Tiss. Organ Cult. 26: 115; Lakshmanan et al., 2006, Plant Cell Rep. 25: 1007) e benzilaminopurina (BAP) (Ali et al., 2008, Pak. J. Bot. 40: 139; Ather et al., 2009, Pak. J. Bot. 41: 815) are efficient sprout inducers. Indolebutyric acid (IBA) (Khatri et al., 2002, Asin J. Plant Sci. 1: 41), gibberellic acid (GA3) (Ather et al., 2009, Pak. J. Bot. 41: 815; Dash et al., 2011, Asian J. Biotechnol. 3: 378) and NAA (Biradar et al., 2009, Karnataka J. Agric. Sci. 22: 21; Ali et al., 2010, Pak. J. Bot. 42: 3783) have been reported for rhizogenesis. Abscisic acid (ABA) is characterized as a growth decelerator, having profound effects on embryogenesis, stimulating, for example, the differentiation of embryogenic calluses (Kaur & Kapoor, 2016). The cytokinin zeatin ((E)-2-methyl-4- (7H-purin-6-ylamino) but-2-en-1-ol) is an inducer of cell division, rupture of apical dominance, neoformation of organs in *in vitro* culture, flowering, and chloroplast development. It is widely used in plant explant regeneration media.

However, some of these compounds, such as benzyladenine (BA) and 2,4-D, address the issue of genetic alterations and abnormal growth in plants grown *in vitro,* while other compounds, such as zeatin, are much more expensive than benzyladenine (BA) and make micropropagation difficult to scale up. In this way, cytokinins that are substitutes for those mentioned above can be used, such as topolins.

Therefore, this invention describes a nutrient substrate comprising at least a basal composition and a supplementation composition that enables the cultivation, germination, regeneration, micropropagation, protection, storage, cryopreservation, hardening and acclimatization of a plant explant and/or plant propagule that has or has not been previously cultivated *in vitro,* giving it metabolic and physiological characteristics such as robustness and root volume, increased leaf area, optimized photosynthetic system, among others, sufficient for it to resist, survive and develop in an *ex vitro* environment under various environmental stresses.

The basal composition of the nutritional substrate of this invention comprises, but is not limited to, basal salts, vitamins, antioxidant agents, carbon sources, adsorbing agents and gelling agents. The supplementation composition of the present invention comprises at least, but is not limited to, a combination of one or more cytokinins with melatonin, and optionally an amino acid composition and a silicon source. Cytokinins are preferentially topolins, more specifically meta-topolin. In addition, the nutrient substrate can contain other compounds commonly used in culture media and synthetic seeds, such as, but not limited to, other phytoregulators, antibiotics, antibacterials, antifungals, nematicides, fertilizers, herbicides, bioinoculants, among others.

The basal salts of the basal composition of the nutrient substrate of the present invention can be any that provide the organic or inorganic micro- and macronutrients necessary for the maintenance, cultivation, and development of a plant explant. Examples of inorganic compounds are Nitrogen (N), Potassium (K), Phosphorus (P), Calcium (Ca), Sulfur (S) and Magnesium (Mg), which are generally required in millimolar quantities. The optimum concentration of each nutrient varies considerably depending on the plant species, type of crop, etc. Plant cells can grow with nitrate (NO⁻³) as the only source of nitrogen, however, there is often a beneficial effect with the addition of ammonium (NH⁴⁺) to the culture medium, making it an essential nutrient for some species. Other sources of reduced nitrogen can also be added to the culture medium. Nitrate is normally used in concentrations between 25 and 40 mM. The amount of ammonium can vary between 2 and 20 mM. Plant cells can be grown in a medium where the only source of nitrogen is ammonium, producing citrate, succinate, malate or another acid from the TCA cycle. It is also possible to grow plant cells with other nitrogen sources, such as urea, glutamine or hydrolyzed casein, among others.

Preferably, the basal salt composition of the nutrient substrate of the present invention is one of MS salts and Hoagland's basal salt (or Hoagland's medium) (Hoagland & Arnon, 1950) with the intention of directing growth and stabilizing the osmotic potential of the medium.

Vitamins are necessary for plant growth and development and are synthesized endogenously by plants. However, when plant cells or tissues are placed in an *in vitro* culture medium, some vitamins become limiting and need to be supplemented. For example, there is a minimum concentration of the vitamin thiamine and increasing it can be used to optimize the growth and development of a plant explant grown *in vitro.* Other vitamins used by plants for growth and which may make up the basal composition of the nutritional substrate of the present invention are nicotinic acid, pyridoxine, calcium pantothenate, biotin, p-amino-benzoic acid, riboflavin, ascorbic acid, citric acid, vitamin B6, folic acidnic acid, among others.

Preferably, the vitamins added to the basal composition of the nutrient substrate are the B5G formulation (Gamborg et al., 1968). The main difference between the widely used MS and B5 vitamin formulations is linked to the different concentrations of the components. For example, vitamin thiamine is 10 times more concentrated in B5 solution. This fact may be linked to the better performance of the nutrient medium supplemented with this vitamin complex because thiamine is an essential cofactor for aerobic respiration enzymes in plants, acting as a greater stimulus for growth and germination (Goyer, 2017).

The antioxidant agents present in the basal composition of the nutrient substrate of the present invention can be any organic or inorganic compound that reacts with the metals present in the culture medium, preventing them from becoming available for oxidation (Matkowski, 2008). Oxidizing agents can be PVP (polyvinylpyrrolidone), which adsorbs the exudates released by the explant which can cause oxidation of the plant material in the early stages of development (Matkowski, 2008); and citric and ascorbic acids, cysteine, nicotinic acid, glutathione, lipoic acid, salicylic acid, tocopherol, polyamines, among others.

The carbohydrate source in the culture media is the immediate supplier of energy for cell growth before the plant acquires autotrophy. In addition, the carbohydrate source influences the development of highly embryogenic cultures and can act as a morphogenic and phytoregulatory agent (Pien et al., 2001).

The carbon sources present in the basal composition of the nutritional substrate of the present invention can be any carbohydrate that can be used and metabolized by plant cells. In this regard, sucrose is the most commonly used carbohydrate in sugarcane micropropagation protocols (Lipavská, H. & Konrádová, H., 2004), although some studies report that replacing sucrose with maltose or corn syrup has positive effects on the embryogenicity of the cultures (Gill et al., 2004; D2, Kaur & Kapoor, 2016). In addition to sucrose and maltose, mannitol stands out for its osmoregulatory properties, which is used in many studies to cause osmotic stress and induce the formation of somatic embryos (George et al., 2008).

Thus, the carbon source of the basal composition of the nutritional substrate of the present invention is preferably sucrose, or conventional sugar, but it can also be glucose, fructose, maltose, or a combination of these. Other carbohydrates have also been tested and can also make up the basal composition, such as lactose, galactose, starch, and sorbitol.

Adsorbing agents are responsible for adsorbing and reducing the availability of exogenous auxin in the culture medium and inducing the process of *in vitro* rhizogenesis (Pan & Van Staden, 1998). They also act to control the effect of phenolic substances released into the culture medium by the explants, which are often related to an increase in the embryogenic potential of these cultures (Kaur & Kapoor, 2016). These compounds can be activated carbon and graphite, for example. Preferably, the basic composition of the nutrient substrate of the present invention comprises activated carbon.

Finally, gelling agents can be all those which, at room temperature, are in a solid/gelled state. Choosing the right gelling agent must take into account several characteristics, including not containing considerable amounts of impurities that will unbalance the chemical and physical properties of the medium; maintaining a complexation that does not interfere with the availability of minerals in the medium and maintaining the "status" of the water in the medium (malic potential). In addition, by applying the IPN (interpenetrating polymer network) concept to the construction of a gelled matrix, by mixing two gelling agents there is greater durability at higher temperatures while at the same time not restricting aeration. The most commonly used gelling agents in culture media are agar, gelatine, pig skin gelatine, bovine gelatine, gelrite, agarose, phytagel, cassava or corn starch, among others. Preferably, for the nutritional substrate of the present invention, the gelling agents are made up of a combination of bacteriological agar and pigskin gelatine.

Thus, the basal composition of the nutritional substrate of the present invention comprises, but is not limited to, the following components in the following concentration ranges:

| **Components (basal composition)** | **Concentration range** |
|---|---|
| *Hoagland's medium* salt | 1.0 to 2.0 g/L |
| Commercial sugar | 20 to 50 g/L |
| Vitamin B5G 1000X | 0.1 to 3 mL/L |
| PVP - 10 | 0.1 to 3.0 g/L |
| Ascorbic acid | 0.10 to 0.50 g/L |
| Citric acid | 0.10 to 0.50 g/L |
| Bacteriological agar | 3 to 25 g/L |
| Pork jelly | 5 to 35 g/L |
| Activated carbon | 0.5 to 3 g/L |

Preferably, the concentration of the components of the basal composition of the nutritional substrate of the present invention can vary according to the following ranges:

| **Components (basal composition)** | **Concentration range** |
|---|---|
| Hoagland's medium salt | 1.50 to 1.70 g/L |
| Commercial sugar | 26 to 36 g/L |
| Vitamin B5G 1000X | 0.5 to 1.5 mL/L |
| PVP - 10 | 0.3 to 1.0 g/L |
| Ascorbic acid | 0.10 to 0.20 g/L |
| Citric acid | 0.10 to 0.20 g/L |
| Bacteriological agar | 7 to 15 g/L |
| Pork jelly | 13 to 25 g/L |
| Activated carbon | 1.0 to 1.75 g/L |

Furthermore, the basic composition of the nutritional substrate of the present invention may include casein hydrolysates, not only as a source of nitrogen, but also as a general adjuvant for growth, induction, maintenance of embryogenic potential and plant regeneration (Gandonou et al., 2005).

In addition, the basal composition may comprise a bioinoculant, biostimulant, fertilizer, phyto-regulator or/and phytohormone, growth-regulating compound (such as auxins, especially but not limited to the auxin 2,4-D, naphthaleneacetic acid (NAA), benzylaminopurine (BAP), indolebutyric acid (IBA), gibberellic acid (GA3), abscisic acid (ABA) and indoleacetic acid (IAA)), antibiotics, antifungals, antibacterials, nematicides, herbicides, among others.

Carrying out the present invention, the basal composition may also comprise rhizogenesis inducers, such as: IAA (indoleacetic acid), phenolic compounds, TIBA (2,3,5-triiodobenzoic acid), synthetic anti-gibberellins and inhibitors of gibberellin biosynthesis, naphthalene acetic acid (ANA) and indolbutyric acid (AIB).

The supplementation composition of the present invention comprises at least one combination of a cytokinin and melatonin, where the cytokinin is preferably a topolin.

According to Aremu *et al.* 2012, it was known that topolins regulated the amount of some secondary metabolites responsible for plant adaptation and survival to environmental variations, such as phenolic compounds. But the type of topolin and its concentration had not yet been indicated, much less in combination with melatonin and at early stages of development, as is the case with the crops of this invention.

In this sense, this invention describes a presente invenção descreve uma supplement composition that comprises, at least, one topolin combined with melatonin. The topolin is one of the meta-topolin(mT), meta-topolin riboside (mTR), meta-methoxytopolin (MemT), meta-methoxytopolin riboside (MemTR), meta-methoxytopolin 9-tetrahydropyran-2-yl (MemTTHP) or any of their agriculturally stable salts.

The concentrations of topolin in the nutrient substrate range from 1 to 15 µM, more preferably from 3.5 to 10 µM, even more preferably the concentration of topolin in the present invention ranges from 4 to 7 µM. More specifically, the topolin concentration ranges from 5 to 6 µM, even more specifically, the topolin concentration is 5 µM.

In addition, melatonin is known to have some important biological effects on plants, such as maximizing/inducing root and shoot growth, activating seed germination and delaying induced leaf senescence. Exogenously, melatonin acts to strengthen plants subjected to heat stress (by inducing the expression of heat shock proteins HSP70 and HSP90) (Arnao & Hernández-Ruiz, 2014; Nawaz et al., 2016). Nawaz et al., 2016 reveals that *Poaceae* and *Brassicaceae* are known to have high concentrations of melatonin in the roots and that its concentration is affected by genotype and environmental factors. In addition, the authors reveal that the concentration of melatonin varies between different cultivars of the same species and is related to the germination of fruit seeds. There are indications that small concentrations of exogenous melatonin increase plants' resistance to water stress by increasing the activity of antioxidant enzymes. For this reason, Nawaz *et al.*, 2011 indicates that a good future strategy would be to cover seeds and fruit with this compound. Arnao & Hernández-Ruiz et al., 2018 describe that the biological function of melatonin is indeed related to the formation of roots and stimulation of the development of the plant area, but that concentrations greater than 10uM of this compound in the roots act in the opposite way, inhibiting plant growth.

However, no state of the art document indicates the best concentration of melatonin to achieve both effects together: stimulation of growth/regeneration and greater adaptability and resistance to stress. Furthermore, it has not been reported that a growing medium already containing cytokinins and other phytoregulators could be supplemented with melatonin to increase/potentiate the physiological characteristics responsible for the growth and resistance of plant explants.

Thus, the supplementation composition of the present invention comprises at least one combination of a cytokinin, preferably a topolin, more preferably a meta-topolin, with melatonin. Preferably, melatonin is present in concentrations ranging from 5 to 25 uM. More preferably, the concentration of melatonin ranges from 8.5 µM to 22.5 µM, even more preferably, the concentration of melatonin ranges from 10 to 20 µM, even more particularly, the concentration of melatonin ranges from 10 µM to 15 µM, and even more specifically, the concentration of melatonin ranges from 10 µM to 15 µM.

In addition, the supplementation composition of the present invention may comprise other compounds, such as, but not limited to, specific amino acids and silicon sources.

The amino acid mixture contributes to the detoxification of reactive oxygen species (ROS), protection of membrane integrity and enzyme/protein stabilization under stress. This mixture can include any of the following: valine, leucine, isoleucine, alanine, arginine, glutamine, lysine, aspartic acid, glutamic acid, proline, cysteine, threonine, methionine, histidine, phenylalanine, tyrosine, tryptophan, asparagine, glycine, serine. In addition, amino acids can come from complex amino acid sources, such as, but not limited to: casein hydrolysates, yeast extract, BSA, amines and organic acids.

For the present invention, it was observed that the combination of the proteinogenic amino acid L-proline, essential for accelerating metabolism and growth (Franzoni et al., 2019), together with L-alanine and glycine enhance the technical effect of the composition for growth supplementation and greater tolerance to stresses in cultivated plant explants.

Finally, the supplementation composition can include a source of silicon, which is a chemical element involved in functions related to the structuring of the cell wall of roots and leaves, thus favoring photosynthesis, increasing the total chlorophyll content and providing more erect leaves, maximizing their architecture due to the accumulation of cellulose, hemicellulose and lignin in the cells (Epstein, 1999).

These characteristics are desirable because they increase survival rates during the acclimatization phase of micropropagated plants, since during this phase there is a greater loss of water due to the low functionality of the stomata and the thin layer of epicuticular wax (Silva, 2007).

In addition, the silicon source acts to reduce transpiration and increase plant resistance to pathogen attacks, as the silicon deposited in the cell wall in the form of amorphous silica can act as a mechanical barrier to fungal invasion.

Thus, the source of silicon in the supplementation composition of the present invention can be any silicate (magnesium, calcium, aluminum, iron, manganese, among others), preferably potassium silicate.

Therefore, in addition to topolin, more specifically meta-topolin and melatonin, the supplement composition can also include, but is not limited to: potassium silicate, proline, alanine, and glycine.

Potassium silicate is present at a concentration ranging from 1 to 10 µM, preferably from 2 to 8 µM, more preferably from 4 to 6 µM. Proline is present at a concentration ranging from 0.1 to 10 mM, preferably from 0.1 to 2 mM, more preferably from 0.2 to 2 mM. Alanine is present at a concentration ranging from 0.05 to 5 mM, preferably from 0.1 to 2 mM, more preferably from 0.1 to 0.8 mM. Glycine is present at a concentration ranging from 0.1 to 4 mM, preferably from 0.3 to 2.5 mM, more preferably from 0.4 to 2 mM.

In one embodiment of the present invention, the nutrient substrate can be placed in any container, tube, wrapping, capsule, etc. for the creation of an artificial seed, in which an explant or plant propagule is placed for cultivation, regeneration, storage, hardening, acclimatization and other purposes. Preferably, any container is filled with the nutrient substrate of the present invention in which a plant propagule is inserted into the nutrient substrate inside said container, forming an artificial seed.

A further embodiment of the present invention comprises a method of cultivating, regenerating, storing, hardening and acclimatizing plant explants or propagules for direct passage to the external *ex vitro* environment, where abiotic stresses may be encountered.

More preferably, the method of the present invention aims to cultivate a plant propagule and comprises the steps of:
(a) *In vitro* cultivation of a plant explant to produce a plant propagule;
(b) insertion of the plant propagule from step (a) into the nutrient substrate of the present invention;
(c) submission of the nutrient substrate from step (b) to *ex vitro* field conditions; and
(d) regeneration of an adult plant.

Accordingly, the present invention describes a nutrient substrate characterized in that it comprises a basal composition for growing plants and a supplementation composition, wherein the supplementation composition comprises at least meta-topolin and melatonin. It also describes a synthetic seed comprising a container filled with the nutrient substrate of the present invention and a plant propagule inserted into the nutrient substrate inside the container. Furthermore, the present invention describes a plant cultivation method comprising the *in vitro* cultivation of a plant explant for the production of a plant propagule and its insertion/placement in the nutrient substrate of the present invention followed by the submission of this nutrient substrate containing the plant propagule to *ex vitro* field conditions for the regeneration of an adult plant. Finally, this invention uses a supplementing composition of the present invention to manufacture a nutrient substrate for growing plants.

"Plant propagule" means any plant tissue that has the capacity or potential to regenerate into an adult plant. Plant propagules can be leaf tissue, meristematic tissue, embryonic tissue, tissue from a true seed, apical buds, root buds, cotyledons, hypocotyls, among others. Preferably, the plant propagule of the present invention is a callus tissue previously cultivated in vitro under callogenesis induction conditions, or an embryonic tissue previously cultivated *in vitro* or not to become an embryogenic tissue.

In the present invention, the plant propagule can be obtained from a Gymnosperm or Angiosperm plant of the monocotyledonous or dicotyledonous class, preferably from a monocotyledonous plant. Even more preferably monocots from the *Poacea* family. Preferably, the plant of interest is a monocot. Of even greater interest, the plant selected is sugarcane (*Saccharum spp.*), which can be natural sugarcane plants, improved varieties, genetically modified sugarcane plants (by genomic editing or conventional transgenics), among others.

A "plant explant" is any plant tissue that has been excised directly from any part of a plant, and differs from "plant propagules" in that it does not necessarily have the inherent capacity to regenerate. For example, a leaf explant is a plant explant according to the present invention, but it is not a plant propagule because it does not yet have the capacity to regenerate into an adult plant directly. Every plant propagule of the present invention is a plant explant, but not every plant explant is a plant propagule according to the present invention.

Regeneration means the process by which a plant propagule or explant develops into an adult plant, either *in vitro* or *ex vitro.*

*"Ex vitro* conditions" means the environmental, water, light and other conditions that are found in environments with little or no control, such as greenhouses, vegetation houses, fields, among others.

### Examples

### Example 1. Testing different ranges of melatonin concentrations to accelerate plant germination or development, optimize root formation and increase resistance to abiotic stresses.

Aggregates of somatic embryos from sugarcane variety 1 from the embryogenic callus induction pathway were used in these experiments. Newly matured somatic embryo clusters were selected for the test of different concentrations of melatonin added to the basal nutrient solution 1 (Table 1)

**Table 1 - Composition of basal medium 1**

| **Reagent** | **Concentration** |
|---|---|
| *Hoagland's medium* salt | 1.63g/L |
| Sucrose | 30g/L |
| Vitamin B5G 1000X | 1mL/L |
| PVP 10 | 0.4g/L |
| Copper sulfate | 0.005g/L |
| Ascorbic acid | 0.15g/L |
| Citric acid | 0.15g/L |
| PPM | 1mL/L |
| Pork skin gelatine | 25g/L |
| Agarose | 8g/L |
| Activated carbon | 1.5g/L |

Treatments 1 to 4 were composed of different concentrations of melatonin: 0, 5, 10 and 30 µM, respectively, microfiltered (0.22 µmol pore) directly into the nutrient solution after autoclaving.

Subsequently, the treatments were poured into trays with 126 wells (0.9 mL each well), where each repetition was made up of a column with 8 wells with a cluster of somatic embryos each, totaling 14 repetitions per tray and a total of 3 trays per treatment. The trays were taken to a controlled growth chamber with a temperature of 28 °C where they remained for 21 days.

The variables analyzed were germination percentage, green area percentage and root volume calculated after 21 days of *in vitro* cultivation using the image analysis system.

After this period, the gelled nutrient substrates of the treatments were placed in plastic containers and stored in the greenhouse to assess survival, shoot length (cm2), leaf area (cm²), number of tillers, chlorophyll a, b and carotenoids after 30 days of *ex vitro* cultivation of the plants from the cultivated somatic embryos.

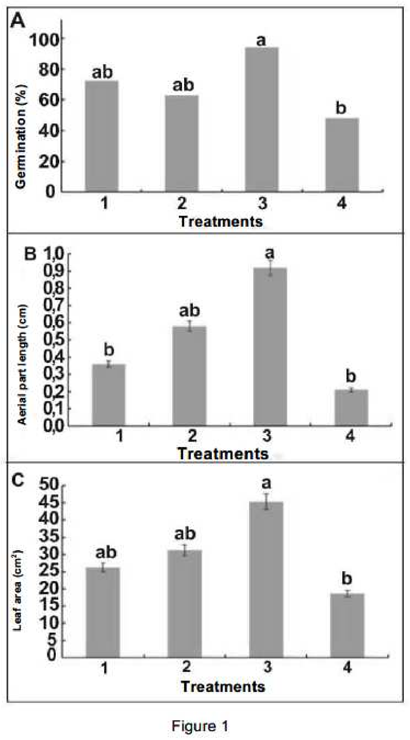

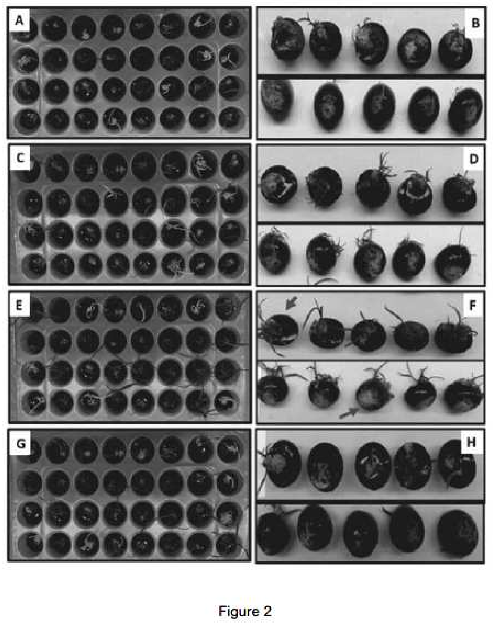

There was a significant difference for the germination variable analyzed after 21 days of *in vitro* cultivation, in which the concentration of 10 µM melatonin (treatment 3) led to a higher germination percentage (90%) (Figure 1C), with greater uniformity in leaf and root growth (Figure 2). For the variables analyzed after 30 days of *ex vitro* cultivation in the greenhouse, shoot length (0.9 cm) (Figure 1B) and leaf area (45 cm²) (Figure 1C) were also higher in treatment 3 (10 µM melatonin).

These results indicate that *in vitro* development was optimized, and the germinated plants showed an increase in adaptive systemic responses to the immediate or future abiotic stresses that will appear in the field with the use of 10 µM melatonin in the supplementation composition.

### Example 2. Optimizing the composition of supplementation with specific amino acids

Regarding the formulation of the basal nutrient solution, Hoagland's basal salt (Hoagland & Arnon, 1950); the vitamin formulation B5G (Gamborg et al., 1968), and the antioxidants PVP (polyvinylpyrrolidone); citric acid and ascorbic acid were previously selected for their superior performance among all the treatments tested previously (they give rise to medium 1). However, the concentration range previously tested for the supplements: aromatic cytokinin meta-topolin; Melatonin (N-Acetyl-5-methoxytryptamine) and amino acids (Proline, Glycine, and Alanine) should be extended to further increase the plants' resistance to simulated water stress in the greenhouse.

The trials used somatic embryo clusters from sugarcane variety 1. Newly mature somatic embryo clusters were selected for the tests.

The formulations of the supplementation composition tested were those described in Table 2 for supplementation of medium 1 (Example 1).

**TABLE 2 - Concentration and components of the composition of the supplement added to the basal medium of nutrient substrate 1.**

| Treatments | Proline (mM) | Alanine (mM) | Glycine (mM) | Melatonin (mM) | Meta-topolin (µM) |
|---|---|---|---|---|---|
| 1 | 0.2 | 0.1 | 0.5 | 10 | 5 |
| 2 | 1 | 0.1 | 0.5 | 10 | 5 |
| 3 | 0.2 | 0.6 | 0.5 | 10 | 5 |
| 4 | 1.5 | 0.6 | 0.4 | 10 | 5 |
| 5 | 0.5 | 0.2 | 1 | 10 | 5 |
| 6 | 1.5 | 0.2 | 1 | 10 | 5 |
| 7 | 0.5 | 0.6 | 1 | 10 | 5 |
| 8 | 1.5 | 0.6 | 1 | 10 | 5 |
| 9 | 0.5 | 0.2 | 0.4 | 20 | 5 |
| 10 | 1.5 | 0.2 | 0.4 | 20 | 5 |
| 11 | 0.5 | 0.6 | 0.4 | 20 | 5 |
| 12 | 1.5 | 0.6 | 0.4 | 20 | 5 |
| 13 | 1 | 0.4 | 2 | 20 | 5 |
| 14 | 1.5 | 0.2 | 1 | 20 | 5 |
| 15 | 0.5 | 0.6 | 1 | 20 | 5 |
| 16 | 1.5 | 0.6 | 1 | 20 | 5 |
| 17 | 0 | 0.4 | 0.7 | 15 | 5 |
| 18 | 2 | 0.4 | 0.7 | 15 | 5 |
| 19 | 1 | 0 | 0.7 | 15 | 5 |
| 20 | 1 | 0.8 | 0.7 | 15 | 5 |
| 21 | 1 | 0.4 | 1 | 15 | 5 |
| 22 | 1 | 0.4 | 1.3 | 15 | 5 |
| 23 | 1 | 0.4 | 0.7 | 5 | 2 |
| 24 | 1 | 0.4 | 0.7 | 25 | 2 |
| 25 | 1 | 0.4 | 0.7 | 15 | 2 |
| 26 Control 1 | 10 | 5 | 2.5 | 5 | 5 |
| 27 Control 2 | 10 | 5 | 2.5 | 10 | 5 |
| 28 Control 3 | 10 | 5 | 2.5 | 20 | 5 |

The *in vitro* experiments consisted of 6 replicates per treatment, where each replicate consisted of a petri dish (100x20) with five somatic embryo clusters each. The variables assessed *in vitro* were: germination percentage; leaf area and root area after 21 days of cultivation using the image analysis system. After this period, the plants from each treatment were individualized and selected into 6 replicates per treatment, with 5 plants in each replicate, transplanted into 25-cell plastic trays filled with moistened substrate (Sphagnum peat pH 5.8 EC 0.3) and stored in the greenhouse to assess survival, shoot length (cm), leaf area (cm²), number of tillers and quantification of chlorophyll a, b and carotenoids after 30 days of *ex vitro* cultivation.

After this period of biometric evaluations in the greenhouse, serial water deficit was started with 96 hours without irrigation followed by hydration (process repeated 4 times), according to Pereira et al. (2005), with adaptations depending on the fraction of water available.

Water stress was monitored using a portable light-modulated fluorometer (Opti-Sciences, model OS1-FL, Hudson, USA), from which the initial fluorescence (F0), maximum fluorescence (Fm), variable fluorescence (Fv) and maximum quantum yield of PSII (Fv/Fm) were measured after the leaves had been adapted to the dark (H"30 min). Measurements were taken before the start of the water deficit period and after each rehydration phase, always between 9 and 11 a.m., thus reflecting the instantaneous status of the photosynthetic apparatus. Two readings were taken on each plant, always in the middle of the +2 leaf.

During the experimental period, the environmental conditions inside the greenhouse, represented by the average temperature and average relative humidity, were measured daily with a hygrograph, between 29.2 +/- 3 °C and 63.4 +/- 5%. The experimental design was entirely randomized. The analysis of variance was carried out using the SISVAR statistical software (Ferreira, 2014), and the means were compared using the Tukey test at 5% probability.

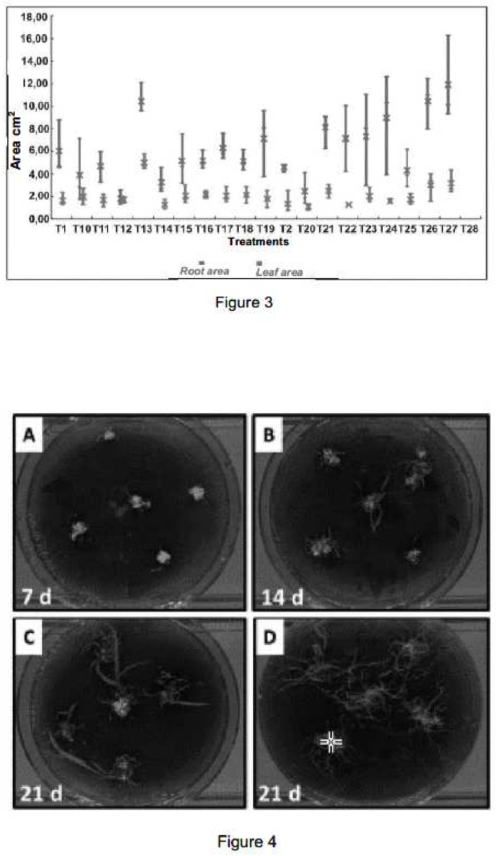

Treatment 13 (1mM proline; 0.4mM Alanine; 2mM Glycine; 20µM melatonin and 5µM meta-topolin) stood out in terms of uniformity between leaf and root area (Figure 3). This result is important because the use of this treatment can optimize the production of synthetic seeds since the somatic embryo aggregates must show root growth, but at the same time efficiency and standardization of the leaf system before insertion into preestablished prototypes. The visual aspect of this treatment is shown in Figure 4.

### Example 4. Optimizing the composition of potassium silicate supplementation

Similarly to Examples 2 and 3, the supplementation solution was also tested for the presence of silicon *in vitro.* The trials used clusters of ES from sugarcane variety 1. Newly matured somatic embryo clusters were selected and previously desiccated. The basal medium tested was medium 1 comprising different concentrations of potassium silicate (K₂SiO₃) (0, 2, 4 and 6 µM) which were microfiltered (pore size 0.22 µmol) directly into the nutrient solution after autoclaving.

Subsequently, the treatments were poured into Petri dishes (100x20), where each repetition consisted of a dish with 10 somatic embryo clusters each, totaling 50 repetitions per concentration. After inoculation, the plates were kept in a conventional growth room, with a 16-hour photoperiod and a light intensity of 82.5 W m⁻² s⁻¹ provided by white, fluorescent lamps, at a temperature of 27 ± 2 °C.

After this period, the germination percentage of the clusters in each treatment was assessed. Subsequently, the plugs were selected and inserted into plastic containers (i.e. artificial seeds) with 20 repetitions in each treatment, planted in 5 L pots in the greenhouse.

Evaluations were carried out at 21, 30, 45 and 60 days and the following parameters were measured: Survival percentage, shoot length (cm), leaf area (cm²), Fv/Fm and quantification of chlorophyll a, b and carotenoids. Root analysis data was obtained using the WinRHIZO root analysis system. Anatomical analyses were performed on leaves +1 collected at 30 and 90 DAP and embedded, and the following anatomical characteristics were analyzed: thickness of the palisade and spongy parenchyma (leaf limb characteristics) under light microscopy.

The treatments tested are described below:

| Treatments | Potassium silicate |
|---|---|
| T1 - control | Medium 1 |
| T2 | 2uM |
| T3 | 4uM |
| T4 | 6uM |

There was a significant difference for the germination variable analyzed after 21 days of *in vitro* cultivation. The concentrations of 2 and 4 µM of K₂SiO₃ did not differ from the control treatment, only the concentration of 6 µM showed phytotoxicity in the ES clusters, causing greater oxidation in the material.

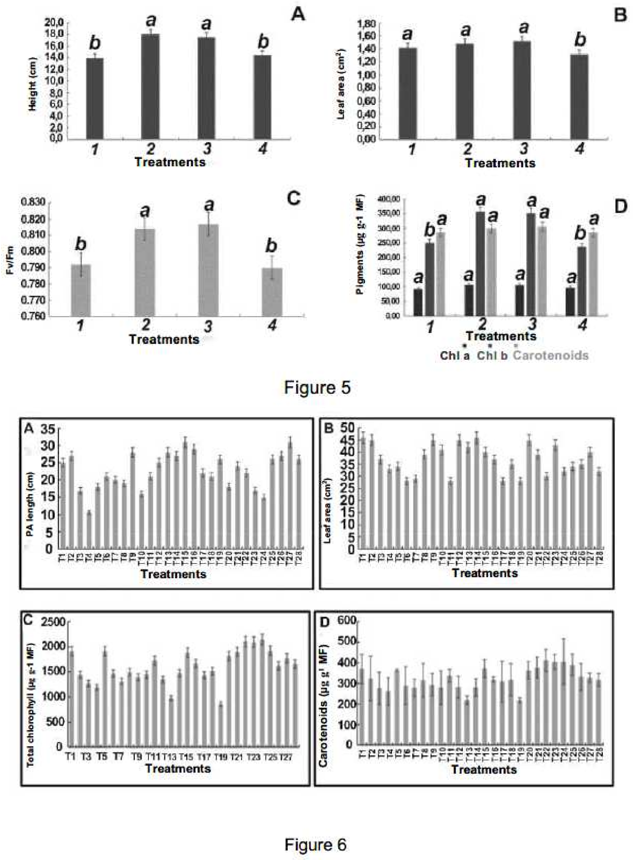

For the survival variable, analyzed 21, 30, 45, 60 and 90 days after planting the artificial seeds in the greenhouse, the material showed a high percentage of survival in treatments 1, 2 and 3 (Figure 5). It can also be seen that treatment 2 showed greater stability in plant survival throughout the evaluations.

However, for the plant height variable, treatment 2 and 3 had the highest averages (Table 3), which can be explained by the fact that the concentrations of K₂SiO₃ tested had a direct impact on the cellular structure of the leaves, which allowed for greater leaf angulation, directly reflecting the shoot length.

**Table 3: Shoot height (cm) at 21, 30, 45, 60 and 90 days after planting the artificial seeds. Averages followed by the same letters do not differ according to the Scott-Knott test (p-value ≤ 0.05).**

| Height (cm) | | | | | |
|---|---|---|---|---|---|
| Treatments | 21 days | 30 days | 45 days | 60 days | 90 days |
| 1 | 9 | 14 | 20.5 | 25 | 40.5 |
| 2 | 12 | 18 | 22.5 | 31.4 | 47.4 |
| 3 | 10.5 | 17.5 | 24.5 | 30.5 | 46.2 |
| 4 | 9 | 14.5 | 19.0 | 24.6 | 36.0 |

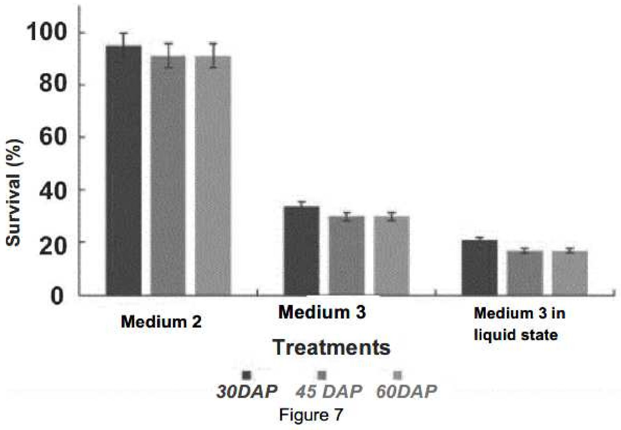

The other parameters were only analyzed at 30 days (Figure 7), and it can be seen that height, leaf area and Fv/Fm differed significantly from the control for treatments 2 and 3.

The results show that treatments 2 and 3, which received concentrations of K₂SiO₃, had higher chlorophyll b levels, which will result in greater adaptability in the field, since variations in the quantity and quality of light are constant, thus favoring the plant's performance

Finally, the results of the root system analysis can be seen in Table 4 below and in Figure 6.

**Table 4 - Data extracted from the WinRhizo system for the variables' length (cm), average caliber (mm) and volume (cm³) of the root system at 90 DAP. The images were colored in the software to make it easier to see the main roots (blue) and secondary roots and root hairs (red and yellow). Averages followed by the same letters do not differ according to the Scott-Knott test (p-value ≤ 0.05).**

| Root system (30 days) | | | |
|---|---|---|---|
| Treatment | Length (cm) | Average caliber (mm) | Volume cm³ |
| 1 | 98.09 *b* | 0.34 *a* | 0.15 *b* |
| 2 | 138.81 *a* | 0.4 *a* | 0.20 *b* |
| 3 | 159.56 *a* | 0.54 *a* | 0.32 *a* |
| 4 | 101.35 *b* | 0.28 *b* | 0.06 *c* |

It can be seen that treatments 2 and 3 had the greatest root lengths, average caliber and volume. The images show that the robustness of the roots can be attributed to the incorporation of silicon into the cells.

As for the anatomical characteristics, it was possible to observe that the palisade parenchyma had an average of one layer in control treatment, two layers in treatment 2 and three to four layers in treatment 3 (data not shown).

The spongy parenchyma showed intercellular spaces characteristic of leaves grown in environments with high humidity. The greater thickness of these leaf limb tissues, such as the parenchyma, gives the plant from the *in vitro* environment a greater chance of survival during transfer to the *ex-vitro* environment, leading to greater photosynthetic activity, which is an important factor for successful transplanting (Braga *et al.*, 2009).

Thus, it can be concluded that the supplementation of 4 µM potassium silicate in the supplementation composition of the present invention enabled a high germination rate *in vitro* and improved the leaf and root structure of the plants, helping to maximize the resistance of the propagules after exposure to *ex vitro* conditions in the field.

### Example 4. Comparison between the nutrient substrate of the present invention with a supplementing composition and without a supplementing composition in the in vitro hardening of sugarcane somatic embryo clusters (ES)

For these experiments, media 2 (medium with the supplementation composition of the present invention) and 3 (medium similar to 2, but without the supplementation composition) were used. Medium 3 represents the formulations commonly reported by the state of the art as being used in plant cell regeneration and/or *in vitro* cultivation processes, while medium 2 of this invention has the supplementation composition composed of L-proline (1 mM), L-alanine (0.4mM), glycine (2mM), the cytokinin meta-topolin (5 µM), melatonin (10 µM) and potassium silicate (3 µM) added to its composition.

| **Medium 3** | |
|---|---|
| **Reagent** | **Concentration** |
| MS salts | 4.3 g/L |
| Sucrose | 30 g/L |
| Vitamin B5G 1,000x | 1 mL/L |
| Glycine | 2mg/L |
| Glutamine | 0.6 g/L |
| Copper sulfate | 0.5 mg/L |
| Activated carbon | 1.5 g/L |
| Citric acid | 0.15 g/L |
| Ascorbic acid | 0.15 g/L |
| Phytagel* | 3 g/L |

| | |
|---|---|
| *Phytagel was only used in gelled medium 3. | |

The trial consisted of three treatments, the first using medium 2, the second using gelled medium 3 and the last using the same medium 3, but in liquid form. (All treatments used clusters of freshly matured and desiccated ES from the RB7515WT sugarcane variety.

Treatments 1 and 2 were poured into Petri dishes (100x20), where each repetition consisted of a dish with 10 clusters of ES each, totaling 30 repetitions per treatment. Treatment 3, on the other hand, was carried out by moistening sterile filter paper with 3-liquid medium on clamshell plates for approximately 21 days. After inoculating the ES clusters, the plates and clamshells were kept in a conventional growth room, with a 16-hour photoperiod and a light intensity of 82.5 W m⁻² s⁻¹ provided by white fluorescent lamps, at a temperature of 27 ± 2 °C.

After this period, the plugs and plants were selected and inserted into predefined containers, with 100 replicates in each treatment, planted in the field in plot 21. Evaluations were carried out at 21, 30, 45 and 60 days and their survival rate was assessed.

It can be seen that the difference between the treatments used was not in terms of the germination of somatic embryo clusters (data not shown), but in terms of the survival of the material, which was assessed 21, 30, 45 and 60 days after planting in the field (Figure 7).

Medium 2 from treatment 1 provided substantially better survival of somatic embryo clusters under stressful field conditions, and the plants regenerated from this material showed greater vigor.

These data show that the supplementation of medium 2 allowed for greater *in vitro* hardening of somatic embryo clusters during the transition from *in vitro* cultivation under controlled conditions to the external environment, where the conditions imposed on plant development are more rigid and require adequate morpho-physiological adaptation to withstand stresses.

It is therefore clear that the inclusion of the supplement composition in nutrient substrate 2 has a superior technical effect compared to substrate 3. This supplementation provided greater *in vitro* hardening and adaptability of the sugarcane somatic embryo clusters when transferred from *in vitro* cultivation directly to the *ex-vitro* environment (field). This is because this supplementation allowed, among other things, the optimization of the formation of a more robust root system, which made it possible to increase systemic resistance against the immediate stresses of in vitro culture and the stresses that were faced *ex vitro,* especially water.

The examples described represent preferred scopes, and it should be understood that the scope of the present invention contemplates other possible variations and is limited only by the content of the claims, including possible equivalents.

## Claims

1. Supplementation composition, **characterized by** the fact that it comprises a combination of meta-topolin and melatonin.

2. Composition according to claim 1, **characterized in that** the concentration of melatonin is 5 to 25 uM, preferably 10 uM to 15 uM, even more preferably the concentration of melatonin is 10uM, and the concentration of meta-topolin is 3.5 to 7uM, preferably 4 to 6 uM, even more preferably the concentration of meta-topolin is 5 uM.

3. Composition according to any one of claims 1 to 2, **characterized in that** it comprises L-alanine, L-proline and glycine and potassium silicate.

4. Composition of nutritive substrate, **characterized by** the fact that it comprises:
a) a supplementation composition as defined in any of claims 1 to 3; and
b) a basal composition of plant cultivation.

5. Nutritive substrate composition according to claim 4, **characterized in that** the basal composition for growing plants comprises basal salts, vitamins, antioxidant agents, sugars, adsorbing and gelling agents.

6. Nutritive substrate composition according to any one of claims 4 to 5, **characterized in that** the basal salts are Hoagland's salt or MS salts; the vitamins are the B5G formulation; the antioxidants are citric and ascorbic acids and polyvinylpyrrolidone (PVP); the sugars are sucrose; the adsorbing agents are activated carbon; and the gelling agents are agar and pork skin gelatine.

7. Synthetic seed, **characterized by** the fact that it comprises:
a) a container filled with a nutrient substrate composition as defined in any of claims 4 to 6; and
b) a plant propagule or plant explant inserted into the nutrient substrate inside the container from step (a).

8. Synthetic seed according to claim 7, **characterized by** the plant propagule or explant being somatic embryos, zygotic embryos, meristematic tissues, true seeds and/or a totipotent plant explant.

9. Synthetic seed according to any one of claims 7 to 8, **characterized in that** the selected plant species is: wheat, barley, corn, rice, oats, forage grasses, peat, miscanthus, sugar cane, beans, soybeans, peas, tomatoes, rapeseed, grapes, potatoes, pineapples, strawberries, oranges, cassava, tobacco and ornamental species.

10. Synthetic seed according to claim 9, **characterized in that** the plant species is a monocot, more preferably sugarcane.

11. Plant growing method, **characterized by** the fact that it comprises:
a) *in vitro* cultivation of a plant explant to produce a plant propagule;
b) insertion of the plant propagule from step (a) into the nutrient substrate, as defined in any of claims 4 to 6;
c) submission of the nutrient substrate from step (b) to *ex vitro* field conditions;
d) regeneration of an adult plant.

12. Use of a supplementation composition as defined in any one of claims 1 to 3, **characterized in that** it is for making a nutritive substrate for growing plants.
